# EUROPEAN PATENT APPLICATION

(11) **EP 2 526 935 A1**
(43) Date of publication of application: **28.11.2012**
(21) Application number: 11004267.8
(22) Date of filing: 24.05.2011
(51) Int. Cl.: A61K 31/16, C07C 243/30, A61P 35/00

(54) **Hydrazidomycins**

(71) Applicant: Oncotest GmbH, 79108 Freiburg (DE)
(72) Inventor: Fiebig, Hein Herbert, Prof. Dr., 79110 Freiburg (DE); Kelter, Gerhard, Dr., 79238 Ehrenkirchen (DE); Maier, Armin, Dr., 79279 Vörstetten (DE); Ueberschaar, Nico, 07745 Jena (DE); Tchize Ndejouong, Basile Le Sage, Dr., H1N 2R4 Montréal Quebec (CA); Ding, Ling, Dr., 07745 Jena (DE); Hertweck, Christian, Prof. Dr., 04105 Leipzig (DE)
(74) Representative: Müller-Boré & Partner Patentanwälte

(57) **Abstract**

The present invention relates to pharmaceutical formulations comprising specific hydrazidomycin compounds. Since said hydrazidomycin compounds show cytotoxic activities, the present invention further relates to said hydrazidomycin compounds for use in medicine, in particular in the treatment of proliferative disorders.

## Description

The present invention relates to pharmaceutical formulations comprising specific hydrazidomycin compounds. Since said hydrazidomycin compounds show cytotoxic activities, the present invention further relates to said hydrazidomycin compounds for use in medicine, in particular in the treatment of proliferative disorders.

The nitrogen-nitrogen bond as found in hydrazines and its derivatives have been known since 1887. Some hydrazines are present in mushrooms, tobacco, bay leaves and soil; others are synthesized for industrial, military, agricultural and medicinal use. In pharmacology, the hydrazines are well known for their impressively broad and diverse biological activities, many of which are clinically used as drugs. Among them, the most important hydrazide types are: neoiscotin, an antibacterial agent also known for its antituberculous and anti-actinomycotic effects, prodresid, used for its antineoplastic effect, the anti-inflammatory agent phenylbutazone, used for its analgesic and antipyretic effects, the antituberculotic drug phtivazid, and isoniazid, a classical antituberculotic drug introduced in 1952, which has played a major role in the worldwide eradication of tuberculous activity. The latter, which is also used as antileprotic agent, represents one of the first antidepressants discovered. In agriculture, thirty to fourty hydrazine derivatives are commercially produced as herbicides, pesticides, insecticides, fungicides or plant growth regulators. Their use in industry for a variety of purposes is well established. Despite the beneficial use of hydrazines, their harmful effects are also high. In fact, many hydrazine analogues were found to exhibit carcinogenic activities in animals. The shock effects of hydrazine poisoning include convulsions, weakness, vomiting, anorexia, weight loss, cardiac failure, and death in man and several species of experimental animals. The controversial properties of hydrazine derivatives have led to a growing and attractive interest in this class of compounds.

The technical problem underlying the present invention is to provide novel hydrazine compounds that can be beneficially used in e.g. medical applications.

The solution to the above technical problem is achieved by the embodiments characterized in the claims.

In particular, highly unusual natural products were discovered, which possess an interesting substituted diacylhydrazide framework. More specifically, new members of alkylhydrazide natural products named hydrazidomycins were isolated from the chloroform extract of a *Streptomyces atratus* culture, and their structures were elucidated by mass spectrometry (MS) and nuclear magnetic resonance (NMR) techniques. The compounds show no antimicrobial activities but display strong to moderate cytotoxic activities and antitumor properties in a panel of human tumor cell lines.

Accordingly, the present invention relates to pharmaceutical compositions comprising at least one hydrazidomycin compound having the general formula (I) and optionally one or more pharmaceutically acceptable carriers, adjuvants, excipients and/or diluents 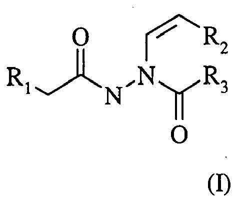 wherein:
R₁ is a group selected from fluorine (F), chlorine (Cl), hydroxy (OH), methoxy (OMe), ethoxy (OEt), propoxy (OPr), iso-propoxy (O-iPr), butyloxy (OBu), iso-butyloxy (O-iBu), tert-butyloxy (O-tBu), phenoxy, benzyloxy, and acetyloxy (OAc); and
R₂ is a group selected from a (C₈-C₂₀)alkyl or (C₈-C₂₀)alkenyl residue, and
R₃ is a group selected from a (C₅-C₁₇)alkyl residue.

The (C₈-C₂₀)alkenyl residue of R₂, which preferably is a (C₁₂-C₁₆)alkyl or (C₁₂-C₁₆)alkenyl residue, can have one or more, particularly two, olefinic unsaturated groups. Generally, the one or more olefinic groups is/are not present at the terminal end. The (C₈-C₂₀)alkenyl residue, preferably (C₁₂-C₁₆)alkenyl residue, can be present in the Z-form or in the E-form or as a mixture thereof.

In a preferred embodiment, R¹ is OCH₃.

In another preferred embodiment R² has 12 or 14 carbon atoms. Most preferably, R² is C₁₂H₂₅, C₁₄H₂₉, C₅H₁₀CH=CHC₇H₁₅ or C₄H₈CH=CHCH₂CH=CHC₅H₁₁.

R₃ is a group selected from a (C₅-C₁₇)alkyl which can be a straight-chain or a branched-chain alkyl residue. Preferably, R₃ is a group selected from a C₉-alkyl residue which more preferably is C₉H₁₉.

In a particular embodiment, the respective compound has the formula (II) and is referred to as hydrazidomycin A:

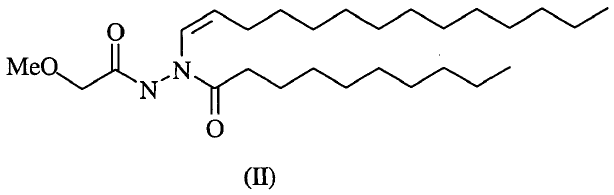

In another particular embodiment, the respective compound has the formula (III) and is referred to as hydrazidomycin B:

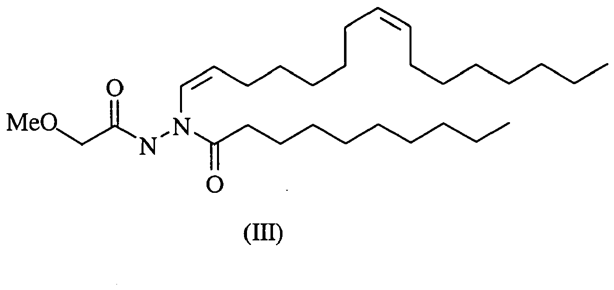

In still another particular embodiment, the respective compound has the formula (IV) and is referred to as hydrazidomycin C:

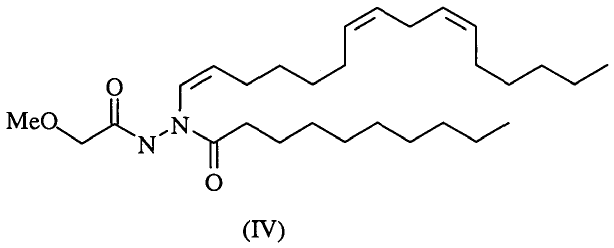

In a preferred embodiment, the hydrazidomycin compounds of the present invention can be conjugated to a biomolecule for target-oriented delivery. Preferably, the respective biomolecule is selected from the group consisting of a peptide, a functional protein, an enzyme, an antigen, an antibody, a monosaccharide, an (oligo)saccharide, a (poly)saccharide, a nucleic acid, a hormone or hormone receptor binder, and a vitamin. The conjugation can be mediated by any suitable linker known in the art.

In another preferred embodiment, the hydrazidomycin compounds of the present invention can be conjugated to molecular moieties that can improve the solubility, penetration, detection, release and/or sensitivity of said compounds. Preferably, the respective molecular moiety is selected from the group consisting of polyethyleneglycols, polyamines, polyguanidines, dyes, receptor ligands, and oligomers as well as polymers. The conjugation can be mediated by any suitable linker known in the art.

In preferred embodiments of the present invention, linkers may comprise an aliphatic chain -(CH₂)ₙ- with n being an integer of from 1 to 12, an oligoethylene glycol -(O-CH₂-CH₂)ₙ- with n being an integer of from 1 to 12, a synthetic poly(ethylene glycol), or a phenylene ring, optionally substituted with one or more substituents selected from the group consisting of F, Cl, Br, 1, NO₂ SO₃H, CN, OH, COOH, a C₁₋₈ alkyl group and a C₁₋₆ aryl group, or combinations thereof. It is particularly preferred that the linker group contains a synthetic polymer selected from the group consisting of poly(ethylene glycol) (PEG), monomethoxy PEG (mPEG), polyglycerol (PG), poly(ethylene imine) (PEI) and N-(2-hydroxypropyl)methacrylamide (HPMA) copolymers, and combinations thereof. It is particularly preferable that the linker group comprises PEG having a mass e.g. ranging from 1,000 to 50,000 Da. It is more preferred that the PEG has a mass in the range of from 2,000 to 20,000 Da. Examples of such linker groups can be derived from commercially available protected mercapto derivatives of PEG of the following structure SuOOC-CH₂-CH₂-PEG-CH₂-CH₂-S-Trt. This group can be converted into a unit for the linker group having the structure -OOC-CH₂-CH₂-PEG-CH₂-CH₂-S-.

Linkers can also contain at any chemically suitable position within the chain a cleavable moiety like a disulfide (reductive or basic-nucleophilic cleavage), an acid or base sensitive ester group (acidic or basic/nucleophilic cleavage, respectively) or an acetal or heteroacetal moiety (acidic cleavage or also oxidative cleavage for *O*/*S* and *S*/*S* acetals), whereby the cleavable moieties may be designed in a way that they respond specifically to certain pH or enzymes that promote such cleavage in vivo.

Accordingly, in the course of the present invention, polyethyleneglycols can serve as linker as well as molecular moieties that can improve solubility, penetration, release or selectivity (e.g. tissue selectivity) of the hydrazidomycin compounds of the present invention.

Further, the present invention relates to hydrazidomycin compounds as defined above for use in medicinal, pharmaceutical, agricultural, biotool or cosmetic applications. In particular, the hydrazidomycin compounds of the present invention can be used in applications which require the killing of cells of any type. Preferably, the hydrazidomycin compounds of the present invention are for use in medicine, more preferably for the inhibition of abnormal cell growth, even more preferably for the treatment of proliferative disorders. In particular, proliferative disorders that can be treated with the hydrazidomycin compounds of the present invention include cancers of the bladder, colon, stomach, head and neck, liver, lung (non-small cell), breast, ovary, pancreas, prostate, kidney, and uterus; melanoma; mesothelioma; sarcoma; leukemia, lymphoma, and myeloma.

In a further aspect, the present invention relates to the use of hydrazidomycin compounds as defined above in the manufacture of a medicament for the treatment of a proliferative disorder. Preferred proliferative disorders are as defined above.

In a final aspect, the present invention relates to a method for the treatment of a proliferative disorder, comprising the step of administering one or more hydrazidomycin compounds as defined above or a pharmaceutical composition as defined above to an individual in need thereof. Preferably, the individual is a human. Further, the individual preferably suffers from a proliferative disorder as defined above.

The figures show:
Figure 1 shows the structures of hydrazidomycins A (II), B (III), and C (IV).
Figure 2 shows selected ¹H, ¹³C-HMBC and ¹H, H-COSY correlations of hydrazidomycins A (II), B (III) and C (IV).
Figure 3 shows the FID plot of the GC/MS analysis of the oxidative cleavage of hydrazidomycin B. The peaks at 7.11 min and 9.49 min correspond to methyl caprylate and methyl decanoate, respectively.

The present invention will be further illustrated in the following examples without being limited thereto.

### Examples

### Experimental procedures:

*General experimental procedures.* Optical rotation was measured using a 0.5 dm cuvette with a Jasco P-1020 polarimeter at 25°C. UV spectra were recorded on a Cary 1 Bio UV-visible spectrophotometer (Varian). IR spectra were recorded on a Bruker FT-IR (IFS 55) spectrometer. All 1 D (¹H, ¹³C , DEPT) and 2D NMR (¹H-¹H COSY, HSQC, NOESY, HMBC) were recorded in deuterated solvents on a Bruker AVANCE II 300, an AVANCE III 500 or 600 MHz instrument equipped with a Bruker Cryo Platform. The chemical shifts are reported in ppm relative to the solvent residual peak (δ ¹H (DMSO) = 2.50 ppm, δ ¹³C (DMSO) = 39.52 ppm). Following abbreviations are used for multiplicities of resonance signals: s = singlet, d = doublet, t = triplet, q = quartet, qt = quintet, m = multiplet, br broad. HRESI-MS were recorded on a Finnigan TSQ quantum ultra (Thermo Fisher Scientific) mass spectrometer. LC-MS measurements were recorded on Agilent 1100 Series LC/MSD Trap. Gas-chromatographic analytics were executed on a Thermo Trace GC Ultra equipped with Combi PAL autosampler and coupled with a FID and a Thermo Polaris Q electron impact (EI) - ion trap mass spectrometer. A SGE forte capillary column BPX5 30 m; 0.25 mm inner diameter and 0.25 µm film was used. The column was operated with helium-carrier gas 1.5 mL/min and split injection (injector temperature 200 °C, detector temperature 250 °C; after initial 1 min at 40°C the oven temperature was raised to 100 °C with 30 °C/min and then to 300 °C with 10 °C/min). Total ion count (TIC) was obtained using the mass range of 50 to 650 amu. The FID temperature was 250 °C. Open column chromatography was performed on silica gel 60 (0.04 to 0.063 mm, 230 to 400 mesh ASTM, Macherey-Nagel) and Sephadex LH-20 (Pharmacia). Pre-coated TLC-sheets Alugram silica gel 60 with fluorescent indicator UV₂₅₄ (Macherey-Nagel) were used. Spots were visualized by spraying with anisaldehyde/sulfuric acid followed by heating. Analytical HPLC was conducted on a Shimadzu SPD-M10A with diode array detector system using a Phenomenex, synergi 4 µ fusion-RP 80 column. Preparative HPLC was performed on a Shimadzu LC-8A with a diode array detector system using also Phenomenex column. All reagents were obtained from commercial suppliers (Sigma Aldrich, TCI, etc.) and used without further purification unless otherwise indicated. All solvents used were spectral grade or distilled prior to use.

*General protocol for oxidation and esterification.* A sample of hydrazidomycin B (2 µL) was dissolved in dichloromethane (1 mL), Aliquat 336 (methyl trioctylammonium chloride, 27 µL) and aqueous potassium permanganate solution (1.7 mL, 2 mg/mL), and the mixture was stirred for 1 h at 8 to 10 °C in an HPLC vial. The solution was diluted with water (10 mL), acidified with sulfuric acid (500 µL) and treated with hydrogen peroxide (100 µL) to decolorize the solution. Dichloromethane (5 mL) was added, and the organic phase was separated and dried over Na₂SO₄. The solution with the free fatty acids was evaporated and treated with N,N-Dimethylformamide dimethyl acetal (DMF-DMA) (150 µL, Applied Science Laboratories, Inc.) in a GC-vial. After 10 min at 140 °C (aluminum block) the mixture was cooled and measured in the GC/MS.

*Taxonomy of the producing strain.* Phenotypic identification of *Streptomyces atratus* was achieved by 16S rDNA analysis (AMODIA Bioservice GmbH, Braunschweig, Germany) and sequence comparison with NCBI data (National Center for Biotechnology Information; NIH, USA) showing a similarity of 98.3%.

*Culture material and fermentation.* The strain was stored in 50% glycerol medium (K₂HPO₄, 6.3 g; citric acid/Na, 0.45 g; MgSO₄-7H_{2O}0, 0.05 g; (NH₄)₂SO₄, 0.9 g; KH₂PO₄, 1.8 g; and glycerol, 44.0 g, in 500 mL distilled water) at -80°C. The strain was grown in 20 mL of Medium 79 (dextrose, 10 g; peptanbacto, 10 g; casamino acids, 1.0 g; yeast extract, 2.0 g; and NaCl, 6.0 g, in 1.0 L distilled water) for 48 hrs at 28°C. Two times 6.0 mL of this culture were used to inoculate two 500 mL Erlenmeyer flasks containing 100 mL of Medium 2 (CaCl₂·2H₂0, 3.0 g; citric acid/Fe III,1.0 g; MnSO₄·H₂O, 0.2 g; ZnCl₂, 0.1 g; CuSO₄·5H₂O, 0.025 g; Na₂B₄O₂·10H₂O, 0.02 g; NaMoO₄·2H₂O, 0.01 g; and oatmeal, 20.0 g, in 1.0 L distilled water). The cultures were shaken on a rotary shaker (180 rpm) at 28 °C for 5 days. Each of these 100 mL cultures was used to inoculate 1.0 L of Medium 2, which was also used for the inoculation of 20.0 L of Medium 2; after 5 days at 28 °C with 180 rpm, the 20 L were finally used as seed culture for 175 L of Medium 2 in a 300 L fermenter vessel. The fermentation was carried out for 6 days with aeration of 25 to 50 L/min, stirring at 200 rpm with a temperature of 28 °C and at a pH range of 5.4 to 6.4.

*Extraction and isolation.* From a large scale fermentation (200 L), mycelium was separated from the culture by filtration and extracted with methanol. The dried extract (110 g) was fixed on silica gel and fractionated through flash chromatography with a gradient CHCl₃/MeOH; CHCl₃ (30 g), CHCl₃/MeOH 1:1 (25 g), MeOH (38 g) and 17 g of waste. The CHCl₃ fraction showed strong cytotoxic activities and was chosen for further separation and purification. This fraction was successively subjected to open column chromatography using silica gel 60 (gradient of CHCl₃/MeOH) and Sephadex LH-20 (eluent MeOH), respectively. Final purification of the hydrazide derivatives was achieved by preparative RP-HPLC (Phenomenex fusion-RP 250 x 21 mm, flow rate 12 mL/min, gradient CH₃CN/H₂O, 60% to 80% in 5 min, 80% to 85% in 10 min and 85% to 100% CH₃CN in 5 min, UV detection at 254 nm) for hydrazidomycin C (26.6 mg), and analytical RP-HPLC (Phenomenex, synergi 4 µ fusion-RP 80, 250 x 4.6 mm, flow rate 1 mL/min, gradient CH₃CN-H₂O, 60% to 80% in 5 min, 80% to 97% in 20 min and 97% to 100% CH₃CN in 5 min, UV detection at 254 nm) for hydrazidomycin B (13.7 mg) and A (2.0 mg).

Hydrazidomycin A: Colorless oil; UV (MeOH) λₘₐₓ: 222 and 201 nm; IR (KBr) cm⁻¹: 3230, 2923, 2853, 2325, 1695, 1465, 1116, and 630; ¹H NMR (600 MHz, DMSO-d₆) and ¹³C NMR (150 MHz, DMSO-D₆) cf. Table 1; HRESI-MS *m*/*z* 453.4029 [M+H]⁺, calcd 453.4051 for C₂₇H₅₂N₂O_{3;} ESIMS-MS daughter ions *m*/*z* 407, 183, 155.

Hydrazidomycin B: Colorless oil; UV (MeOH) λₘₐₓ: 222 and 202 nm; IR (KBr) cm⁻¹: 3230, 2923, 2853, 2325, 1695, 1465, 1116, and 630; ¹H NMR (500 MHz, DMSO-d₆) and ¹³C NMR (125 MHz, DMSO-D₆) cf. Table 1; HRESI-MS *m*/*z* 479.4198 [M+H]⁺, calcd 479.4207 for C₂₉H₅₅N₂O_{3;} ESIMS-MS daughter ions *m*/*z* 433, 183, 155.

Hydrazidomycin C: Colorless oil; UV (MeOH) λmax: 222 and 201 nm; IR (KBr) cm⁻¹: 3231, 2924, 2848, 2325, 1697, 1463, 1115, and 630; ¹H NMR (600 MHz, DMSO-d₆, ¹³C NMR (150 MHz, DMSO-d₆) and ¹⁵N NMR (500 MHz, DMSO-D₆) cf. Table 1; HRESI-MS *m*/*z* 477.4043 [M+H]⁺, calcd 477.4051 for C₂₉H₅₂N₂O_{3;} ESIMS-MS daughter ions *m*/*z* 431, 183, 155.

**Table 1: ¹H NMR and ¹³C NMR data of hydrazidomycin A, B, and C in DMSO-D₆.**

| | **Hydrazidomycin A (II)** | | **Hydrazidomycin B (III)** | | **Hydrazidomycin C (IV)** | |
|---|---|---|---|---|---|---|
| | **δ ¹H in ppm (J/Hz)** | **δ ¹³C in ppm** | **δ ¹H in ppm (J/Hz)** | **δ ¹³C in ppm** | **δ ¹H in ppm (J/Hz)** | **δ ¹³C in ppm** |
| 1 | 3.35, s | 59.0 | 3.35, s | 59.0 | 3.35, s | 59.0 |
| 2 | 3.97, s | 70.7 | 3.97, s | 70.7 | 3.97, s | 70.7 |
| 3 | - | 167.8 | - | 167.7 | - | 167.7 |
| 4 | - | 173.3 | - | 173.2 | - | 173.2 |
| 5 | 2.30, t (7.2) | 31.2 | 2.30, t (7.4) | 31.3 | 2.30, t (7.3) | 31.2 |
| 6 | 1.44, m | 23.9 | 1.44, m 1.44. m | 23.9 | 1.44, m | 23.9 |
| 7 | 1.23, m^{a} | 28.8, 28.7, 28.8, 29.0, 29.1, 29.1,29.2^{b} | 1.23. m^{a} | 28.6, 28.7, 28.8, 28.9, 29.1^{b} | 1.23, m^{a} | 28.5, 28.6, 28.8, 28.9^{b} |
| 8 | 1.23, m^{a} | | 1.23, m^{a} | | 1.23, m^{a} | |
| 9 | 1.23, m^{a} | | 1.23, m^{a} | | 1.23, m^{a} | |
| 10 | 1.23, m^{a} | | 1.23, m^{a} | | 1.23, m^{a} | |
| 11 | 1.20, m^{a} | 31.2 | 1.20, m^{a} | 31.2 | 1.20, m^{a} | 31.1 |
| 12 | 1.23, m^{a} | 22.2 | 1.23, m^{a} | 22.1 | 1.23, m^{a} | 22.2 |
| 13 | 0.84, t (6.9)^{a} | 14.1 | 0.85, t (6.9)^{a} | 13.9 | 0.85, t (6.9)^{a} | 13.9 |
| 1' | 6.41, dt (9.2, 1.6) | 124.3 | 6.41, dt (9.3, 1.6) | 124.3 | 6.41, dt (9.3, 1.7) | 124.3 |
| 2' | 4.76, dt, (9.2, 7.2) | 117.2 | 4.76, dt (9.2, 7.2) | 117.1 | 4.76, dt (9.2, 7.4) | 117.1 |
| 3' | 2.01, m | 25.9 | 2.01, m | 25.9 | 1.23. m^{a} | 28.5. 28.6. 28.8, 28.9^{b} |
| 4' | 1.23, m^{a} | 29.1 | 1.23, m^{a} | 28.6, 28.7, 28.8, 28.9, 29.1^{b} | 1.23, m^{a} | 25.9 |
| 5' | 1.23, m^{a} | 28.6,28.7 28.8, 29.0, 29.1, 29.1,29.2^{b} | 1.23, m^{a} | | 2.01, m | 26.7 |
| 6' | 1.23, M^{a} | | 1.23, m^{a} | | 1.96, m^{a} | 129.7 |
| 7' | 1.23, m^{a} | | 1.96, m^{a} | 26.6 | 5.31, m^{a} | 127.7 |
| 8' | 1.23. m^{a} | | 5.31. t (4.7)^{a} | 129.6 | 5.31, m^{a} | 25.2 |
| 9' | 1.23, m^{a} | | 5.31, t (4.7)^{a} | 129.6 | 2.73, t (6.5) | 127.7 |
| 10' | 1.23, m^{a} | | 1.96, m^{a} | 26.6 | 5.31, m^{a} | 129.7 |
| 11 | 1.23, m^{a} | | 1.23, m^{a} | 28.6, 28.7, 28.8, 28.9, 29.1^{b} | 5.31, m^{a} | 26.6 |
| 12' | 1.23, m^{a} | 31.4 | 1.23, m^{a} | | 1.97, m^{a} | 25.9 |
| 13' | 1.23, m^{a} | 22.2 | 1.23. m^{a} | | 1.23, m^{a} | 28.5. 28.6. 28.8. 28.9^{b} |
| 14' | 0.84, t (6.9)^{a} | 14.1 | 1.23, m^{a} | 31.2 | 1.23, m^{a} | 31.2 |
| 15' | - | - | 1.23, m^{a} | 22.1 | 1.23, m^{a} | 22.1 |
| 16' | - | - | 0.85, t (6.9)^{a} | 13.9 | 0.85, t (6.9)^{a} | 13.9 |
| NH | 10.69, s | - | 10.69, s | - | 10.69, s | - |

| | | | | | | |
|---|---|---|---|---|---|---|
| ^{a} signals are overlapped, signals are not distinctly allocatable | | | | | | |

*Antiproliferative and cytotoxic assays.* A modified propidium iodide assay was used to determine the cytotoxic activity of compounds hydrazidomycin A, B, and C against 12 to 42 cell lines derived from solid human tumors. The test procedure has been as is known in the art. Cell lines tested were derived from patient tumors engrafted as a subcutaneously growing tumor in NMRI nu/nu mice, or obtained from the American Type Culture Collection, Rockville, MD, USA, National Cancer Institute, Bethesda, MD, USA, or Deutsche Sammlung von Mikroorganismen und Zellkulturen, Braunschweig. The IC₅₀ was determined by four parameter non-linear curve fit after plotting compound concentration versus fluorescence intensity, and is given as the concentration of test compound that gives a response (inhibition of cell growth) half way between the top and bottom plateau of the sigmoidal concentration-response curve (*i.e*. the inflection point of the curve).

### Example 1: Isolation of hydrazidomycins A, B, and C

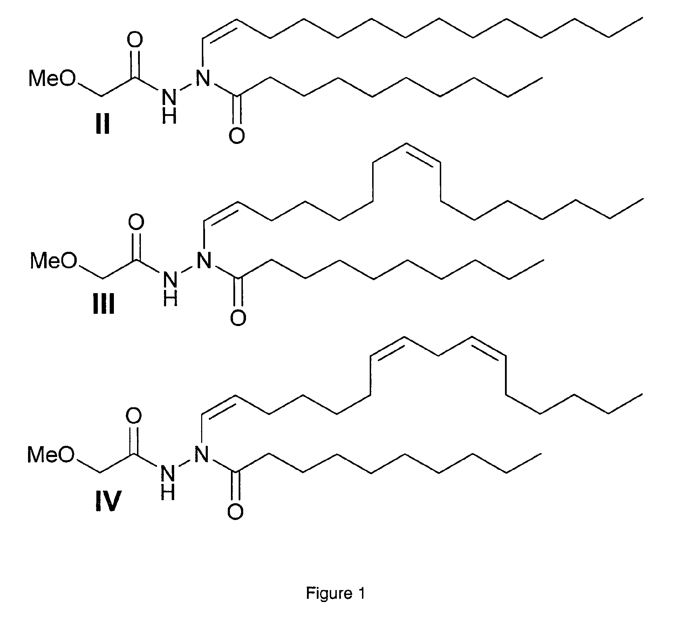

In the search for novel antitumor compounds, the extreme and unusual habitats of the Yunnan province in the southwest of China were investigated for their unique biological and chemical diversity. Out of 36 actinomycetes strain isolates, *Streptomyces atratus* was among those selected for the high antitumor activity of their organic extracts. The methanol extracts of cultures of the *S*. *atratus* strain displayed strong cytotoxic activities against all six tumor cell lines of the screening panel which differ in chemosensitivity toward standard chemotherapeutic agents (gastric cancer GXF 251 L, non-small cell lung cancer LXFL 529L, breast cancer MAXF 401 NL, melanoma MEXF 462NL, renal cancer RXF 486L, uterus cancer UXF 1138L). LC-DAD and MS analyses of a small culture (100 mL) revealed several unknown secondary metabolites in the metabolic profiles. From an up-scaled fermentation (200 L), the harvested mycelium was extracted with MeOH, and the dried extract was fractionated by flash chromatography using a CHCl₃/MeOH gradient. The CHCl₃ fraction, which showed strong cytotoxic activities, was subjected to open column chromatography on silica gel 60 and Sephadex LH-20, respectively. Three homologous products (Figure 1), hydrazidomycin A (1, 2.0 mg) hydrazidomycin B (2, 13.7 mg), and hydrazidomycin C (3, 26.6 mg) were purified by preparative RP-HPLC (for hydrazidomycin C) and analytical RP-HPLC (for hydrazidomycins A and B).

### Example 2: Characterization of hydrazidomycins A, B, and C

The structures of hydrazidomycins A, B, and C were fully characterized by MS experiments and NMR spectroscopy, and the positions of the double bonds were determined through NMR together with ESIMSⁿ experiments and chemical degradation.

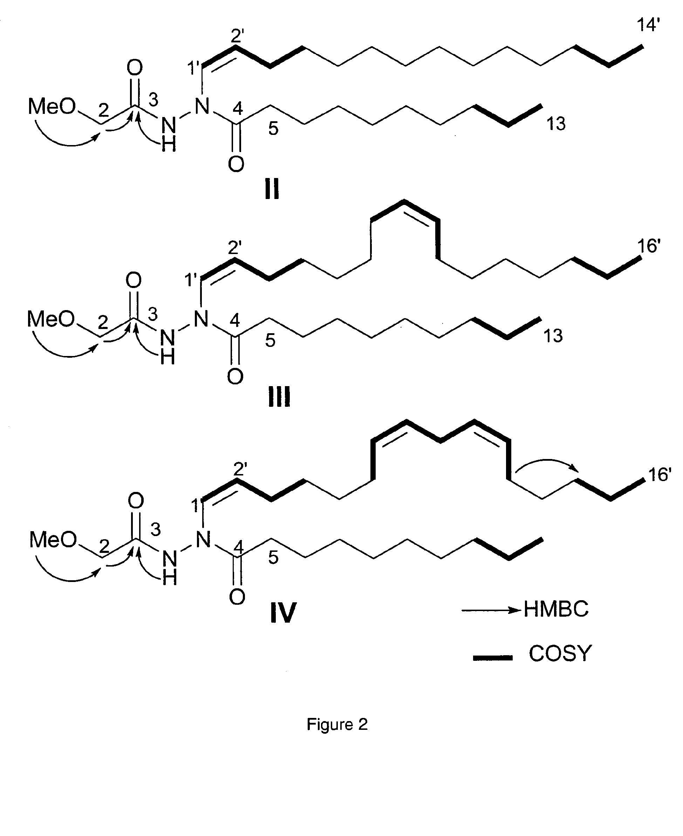

For hydrazidomycin A, an oily substance, the molecular formula of C₂₇H₅₂N₂O₃ was deduced from HRESI-MS (*m*/*z* 453.4040 [M+H]⁺). The presence of two carbonyls (δ 173.2, C-4, 167.7, C-3), two olefinic carbons (δ 124.2, and 117.1), one oxygenated methyl (δ 59.0, C-1), one oxygenated methylene (δ 70.7, C-2), as well as another 17 aliphatic carbons was revealed by inspection of ¹³C NMR, DEPT and HSQC spectra. ¹H NMR and COSY spectra indicated the presence of one NH group (δ 10.69, s), two olefinic protons (δ 6.41, d, δ 4.76, dt), one methoxy (δ 3.35), one oxygenated methylene (δ 3.97), one methylene (δ 2.30, t) adjacent to the carbonyl group, and various aliphatic protons. A 2-methoxyacetamide fragment was readily deduced by HMBC correlations (Figure 2). As no nitrogen bearing methylene was suggested by NMR data, a NH-N-CO connection was proposed. Furthermore, the existence of only one NH further indicated the connection of the double bond to the second nitrogen atom. The COSY spectrum gave evidence for two alkyl chains. To establish the chain length and double bond positions, MS/MS fragmentation experiments were carried out. Fragments with *m*/*z* 155 and *m*/*z* 183 established the shorter chain as a ten carbon chain. With the fragment of *m*/*z* 364 revealed the size of the second chain (14 carbon atoms). The *Z* configuration of the double bond at C-1'/C-2' was deduced from the low coupling constant (*J*= 9.31 Hz) between the olefinic protons H-1'/H-2'.

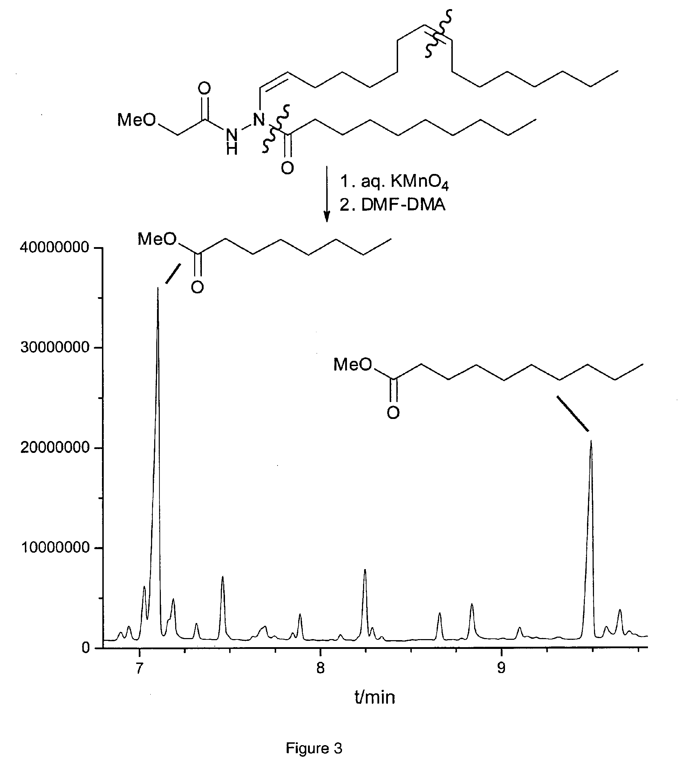

Comparison of the NMR spectra of hydrazidomycins A and B revealed some similarities of these two metabolites. For hydrazidomycin B, the molecular formula of C₂₉H₅₄N₂O₃ was derived from HRESI-MS ([M+H]⁺ 479.4198) which indicated that this compound had two carbon atoms and one double bond equivalent more compared to hydrazidomycin A. The ¹³C NMR spectrum showed signals for the conserved olefinic carbons (δ 124.2, and 117.1) for C-1' and C-2', and signals for two additional carbons (δ 129.6, 129.6) were detected that rationalized the additional double bond equivalent. The 2-methoxyacetamide fragment was readily deduced by HMBC correlations (Figure 2). By MS/MS fragmentation, the fragment with *m*/*z* 155 confirmed the presence of the saturated carbon chain as in hydrazidomycin A. Based on the chemical shifts of allylic methylene carbon signals which were relatively upfield (δ 28.9, 26.6), a *Z* configuration for the C-8'/C-9' double bond was proposed. The position of the additional double bond was determined by MS and chemical degradation. Fragmentation of *m*/*z* 390 resulted in a major fragment with *m*/*z* 306, suggesting that the additional double bond is positioned at C8'. This was confirmed by reaction of hydrazidomycin B with aqueous potassium permanganate under phase transfer conditions, followed by methylation with DMF-DMA, and GC-MS analysis. It was possible to detect the predicted methylesters of C₈ and C₁₀ aliphatic acids, respectively (Figure 3). In addition to verifying the position of the double bond, this result confirmed the presence of the decanoyl moiety.

For hydrazidomycin C, which was also isolated as an oily substance, a molecular formula of C₂₉H₅₂N₂O₃ (calcd 477.4051) was deduced from HRESI-MS (*m*/*z* 477.4043 [M+H]⁺) and ¹³C NMR data. Accordingly, hydrazidomycin C would share the same number of carbons with hydrazidomycin B, yet it has one additional double bond equivalent. The presence of six olefinic carbons (δ 129.6, 129.6, 127.7, 127.7, 124.2, and 117.1) was revealed by inspection of ¹³C NMR, DEPT and HSQC spectra. ¹H NMR and COSY spectra indicated the presence of one NH group (δ 10.69), six olefinic protons, one methoxy group (δ 3.35), one oxygenated methylene (δ 3.97), four allylic protons, including one characteristic diallylic methylene (δ_{H} 2.71), and unresolved signals of aliphatic protons. Like hydrazidomycins A and B, hydrazidomycin C features the 2-methoxyacetamide fragment. To elucidate the chain length of the fatty acid and alkyl residues and to determine the double bond positions, MS/MS fragmentation experiments were carried out. A fragment with *m*/*z* 155 showed that hydrazidomycin C has the same alkenyl substituent as hydrazidomycins A and B. Furthermore, fragmentation of *m*/*z* 388 resulted in a major fragment with *m*/*z* 304, which indicated the position of the skipped double bonds. The Z configuration for the C-6'/C-7' and C-9'/C-10' double bonds was assigned on the basis of the relative upfield chemical shifts of the allylic methylene carbon signals (δ 26.7, 26.6).

Hydrazidomycins A, B, and C bear an unusual trisubstituted hydrazide core and only vary in the type of fatty acid chains. Notably, only a limited number of natural products possess the unusual hydrazide diamide, with montamine and 4-methyl-1,2,6,8-tetraazacycloundeca-4,9-diene-3,7,11-trione being rare examples.

### Example 3: Biological activities of hydrazidomycins A, B, and C

Hydrazidomycins A, B, and C were evaluated for their antimicrobial and antitumor activities. Overall, said compounds showed no antimicrobial activity against *Bacillus subtilis, Staphylococcus aureus, Escherichia coli, Pseudomonas aeruginosa, Staphylococcus aureus, Enterococcus faecalis* and *Mycobacerium* vaccae, respectively, at a concentration of 1.0 mg/mL, but displayed moderate to strong cytotoxic effects (cf. Table 2), with hydrazidomycin A being the most effective one (mean IC₅₀ = 0.37 µM). Hydrazidomycin C did not show inhibition of tumor cell growth up to a concentration of 10 µM.

**Table 2: Cytotoxicity of hydrazidomycins in a panel of 12 human tumor cell lines. ^{a}geometric mean IC₅₀ [µM]; ^{b}number of cell lines with IC₅₀ ≤ ½ geometric mean IC₅₀ over total number of cell lines.**

| **Compound** | **Cytotoxic potency^{a}** | **Above average activity^{b}** |
|---|---|---|
| Hydrazidomycin C | > 10.00 | 0/12 (0%) |
| Hydrazidomycin B | 6.04 | 2/12 (17%) |
| Hydrazidomycin A | 0.37 | 1/12 (8%) |

Hydrazidomycins A and B were subjected to more detailed investigations in a panel of 42 tumor cell lines representing 15 different histotypes. The initial findings could be reproduced as the compounds displayed moderate to strong cytotoxic effects with mean IC₅₀ values of 0.86 µM (hydrazidomycin A) and 10.7 µM (hydrazidomycin B) (cf. Table 3). Hydrazidomycin A (1) was identified to be more potent compared to hydrazidomycin B (2). Differential antitumor activity, as expressed by the deviation of individual IC₅₀ values from the mean IC₅₀ value against all cell lines tested, was observed for both compounds with above average activity in 6/42 (14.3%) cell lines (hydrazidomycin A), or in 4/42 (9.5%) cell lines (hydrazidomycin B). Hydrazidomycins A and B were most active in cell lines of stomach cancer, prostate cancer, non small cell lung cancer, and breast cancer. Hydrazidomycin A was further active in cell lines of colon cancer and liver cancer. IC₅₀ values in these sensitive cell lines were on average about 4-fold (hydrazidomycin A), or 8-fold (hydrazidomycin B) lower than the mean IC₅₀ of all cell lines tested. Apparently, the cytotoxicity of the hydrazides decreases as the number of double bonds of the fatty acid chain increases, suggesting that the rigidity of the fatty acid chain reduces the cytotoxicity.

**Table 3: Cytotoxicity of hydrazidomycins A (II) and B (III) in a panel of 42 human tumor cell lines.**

| **Histotype** | **Cell line** | **IC₅₀ (µM)** | |
|---|---|---|---|
| | | **(II)** | **(III)** |
| **Bladder** | BXF 1218L | 2.43 | 19.3 |
| | BXF 1352L | 2.30 | 15.1 |
| | T-24 | - | 9.19 |
| **Colon** | CXF 269L | 0.222 | 5.94 |
| | DIFI | 0.782 | 10.2 |
| | HCT-116 | 0.758 | 19.4 |
| | HT-29 | 0.464 | 6.54 |
| | RKO | 0.598 | 13.8 |
| **Stomach** | MKN45 | 0.676 | 11.6 |
| | GXF 251L | 0.144 | 1.54 |
| **Head and neck** | CAL-27 | 0.716 | 12.7 |
| **Liver** | LIXF 575L | 0.349 | 7.39 |
| **Lung, non-small cell** | LXFA 289L | 1.10 | 12.6 |
| | LXFA 526L | 1.65 | 7.07 |
| | LXFA 629L | 0.247 | 1.41 |
| | LXFL1121L | 2.31 | 17.4 |
| | LXFL 529L | 2.12 | 17.3 |
| | NCI-H460 | 2.31 | 17.2 |
| **Breast** | MAXF 401NL | 0.471 | 0.876 |
| | MCF-7 | 0.782 | 11.0 |
| | MDA-MB-231 | 0.286 | 5.53 |
| **Melanoma** | MEXF 1341L | 0.936 | 14.9 |
| | MEXF 276L | 1.59 | 18.3 |
| | MEXF 462NL | 0.765 | 20.4 |
| **Ovary** | OVXF 899L | 1.34 | 19.1 |
| | NIH:OVCAR-3 | 1.10 | 18.5 |
| **Pancreas** | PAXF 1657L | 1.91 | 20.4 |
| | PAXF 546L | 0.660 | 10.8 |
| | PANC-1 | 0.933 | 10.0 |
| **Prostate** | 22Rv1 | 0.882 | 15.6 |
| | DU-145 | 0.539 | 7.22 |
| | LNCaP | 0.957 | 14.1 |
| | PC-3M | 0.105 | 1.73 |
| **Mesothelioma** | PXF 1118L | 2.40 | 30.0 |
| | PXF 1752L | 1.19 | 12.7 |
| | PXF 698L | 0.982 | 14.3 |
| **Kidney** | RXF 1781L | 0.982 | 10.2 |
| | RXF 393NL | 1.11 | 11.0 |
| | RXF 486L | 1.62 | 21.2 |
| **Sarcoma** | Saos-2 | 1.82 | 18.0 |
| | TE671 | 0.898 | 18.2 |
| **Uterus** | UXF 1138L | 0.893 | 18.2 |
| **mean IC₅₀** | | 0.857 | 10.7 |

## Claims

1. Pharmaceutical composition comprising at least one hydrazidomycin compound having the general formula (I) and optionally one or more pharmaceutically acceptable carriers, adjuvants, excipients and/or diluents, 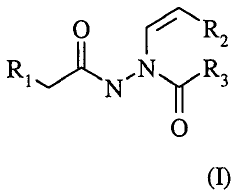 wherein:
R₁ is a group selected from fluorine (F), chlorine (Cl), hydroxy (OH), methoxy (OMe), ethoxy (OEt), propoxy (OPr), iso-propoxy (O-iPr), butyloxy (OBu),
iso-butyloxy (O-iBu), tert-butyloxy (O-tBu), phenoxy, benzyloxy, and acetyloxy (OAc); and
R₂ is a group selected from a (C₈-C₂₀)alkyl or (C₈-C₂₀)alkenyl residue, and
R₃ is a group selected from a (C₅-C₁₇)alkyl residue.

2. The pharmaceutical composition according to claim 1, wherein the (C₁₂-C₁₆)alkenyl residue has one or two olefinic unsaturated groups.

3. The pharmaceutical composition according to claim 1 or 2, wherein R² has 12 or 14 carbon atoms.

4. The pharmaceutical composition according to claim 1 or 2, wherein R² is C₁₂H₂₅, C₁₄H₂₉, C₅H₁₀CH=CHC₇H₁₅ or C₄H₈CH=CHCH₂CH=CHC₅H₁₁.

5. The pharmaceutical composition according to any one of claims 1 to 4, wherein the hydrazidomycin compound has the following formula (II):

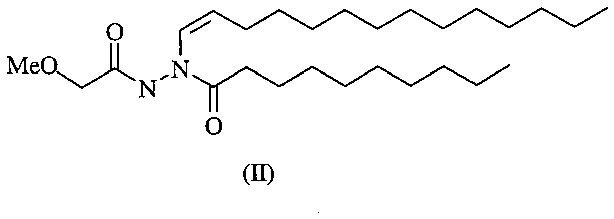

6. The pharmaceutical composition according to any one of claims 1 to 4, wherein the hydrazidomycin compound has the following formula (III):

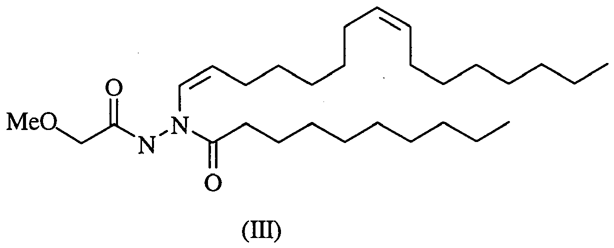

7. The pharmaceutical composition according to any one of claims 1 to 4, wherein the hydrazidomycin compound has the following formula (IV):

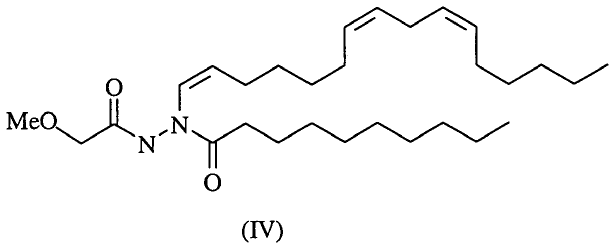

8. The pharmaceutical composition according to any one of claims 1 to 7, wherein the hydrazidomycin compound is conjugated to
(i) a biomolecule for the target-oriented delivery, or
(ii) a molecular moiety which can improve their solubility, penetration, detection, release or selectivity.

9. The pharmaceutical composition according to claim 8, wherein the biomolecule is selected from the group consisting of a peptide, a functional protein, an enzyme, an antigen, an antibody, an (oligo)saccharide, a (poly)saccharide, a nucleic acid, a hormone or hormone receptor binder, and a vitamin.

10. The pharmaceutical composition according to claim 8, wherein the molecular moiety is selected from the group consisting of polyethyleneglycols, polyamines, polyguanidines, dyes, receptor ligands, and polymers.

11. Hydrazidomycin compound having the general formula (I): 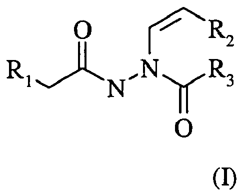 wherein:
R₁ is a group selected from fluorine (F), chlorine (Cl), hydroxy (OH), methoxy (OMe), ethoxy (OEt), propoxy (OPr), iso-propoxy (O-iPr), butyloxy (OBu),
iso-butyloxy (O-iBu), tert-butyloxy (O-tBu), phenoxy, benzyloxy, and acetyloxy (OAc); and
R₂ is a group selected from a (C₈-C₂₀)alkyl or (C₈-C₂₀)alkenyl residue, and
R₃ is a group selected from a (C₅-C₁₇)alkyl residue,
for use in medicine.

12. The hydrazidomycin compound according to claim 11 for use in the treatment of proliferative disorders.

13. The hydrazidomycin compound according to claim 12, wherein the proliferative disorder is selected from the group consisting of cancers of the bladder, colon, stomach, head and neck, liver, lung (non-small cell), breast, ovary, pancreas, prostate, kidney, and uterus; melanoma; mesothelioma; sarcoma; leukemia, lymphoma, and myeloma.

14. Hydrazidomycin compound having the following formula (II), (III) or (IV):
